(19) Europäisches Patentamt
European Patent Office
Office européen des brevets

(11) **EP 0 643 881 B1**

(12) **EUROPEAN PATENT SPECIFICATION**

(45) Date of publication and mention
of the grant of the patent:
**16.12.1998   Bulletin 1998/51**

(51) Int Cl.[6]: **H03B 29/00**, G10K 11/16

(21) Application number: **92913700.8**

(22) Date of filing: **05.06.1992**

(86) International application number:
**PCT/US92/04567**

(87) International publication number:
**WO 93/26084 (23.12.1993 Gazette 1993/30)**

(54) **ACTIVE PLUS SELECTIVE HEADSET**

AKTIVER KOPFHÖRER MIT ERHÖHTER SELEKTIVITÄT

CASQUE D'ECOUTE ACTIF ET SELECTIF

(84) Designated Contracting States:
**AT BE CH DE DK ES FR GB GR IT LI LU MC NL SE**

(43) Date of publication of application:
**22.03.1995   Bulletin 1995/12**

(73) Proprietor: **NOISE CANCELLATION
TECHNOLOGIES, INC.
Linthicum, MD 21090 (US)**

(72) Inventors:
• **CLAYBAUGH, David
Germantown, MD 20874 (US)**
• **DENENBERG, Jeffrey
Trumbull, CT 06611 (US)**
• **BUSCH, Ralph
Takoma Park, MD 20912 (US)**

• **HOHMAN, John
Towson, MD 21204 (US)**

(74) Representative: **Wilhelm & Dauster
Patentanwälte European Patent Attorneys
Hospitalstrasse 8
70174 Stuttgart (DE)**

(56) References cited:
US-A- 3 952 158          US-A- 4 061 158
US-A- 4 064 362          US-A- 4 677 678
US-A- 4 953 217          US-A- 5 046 103
US-A- 5 091 953          US-A- 5 105 377

• **IEEE POTENTIALS, vol. 11, no. 2, April 1992,
USA, J. N. DENNENBERG 'Anti-noise: Quieting
the environment with active noise cancellation
technology'**

# Description

This invention relates to a headset according to the introductory part of claim 1.

In the past, attempts to combine the two protections i.e., high and low frequency attenuation, has resulted in not only the noise being attenuated but also the speech that the wearer needs to hear. Some systems met only limited success with fixed or "near-stationary" noise but not with the other noise of either (a) varying spectral characteristics or (b) brief duration noises with "spikes". Examples of such a system is found in US-patent no. 4 025 721 and US-patent 4 185 168. Other systems like that found in US-patent no. 4 455 675 actively attenuate all sounds at low frequencies and passively attenuate all high frequency sounds. These sounds include speech and warning signals that want to be heard by the person wearing the headset.

In applications for noise canceling headsets, particularly in industrial environments, attenuation of low frequency noise as well as noise that covers the speech band (300 to 3300 Hz) passive hearing protection works extremely well at higher frequencies (typically above 1000 Hz) whereas active noise cancellation has been shown to achieve similar levels of protection at low frequencies (50 to 1000 Hz). Passive, however, also attenuates speech and warning signals and the protectors are uncomfortable to wear.

The object of the present invention is to provide a headset system for actively cancelling unwanted noise while selectively allowing necessary speech to reach the user's ear, a handset means adapted to be worn by user, means for attenuating noise, and speech filtering means for actively cancelling unwanted noise while selectively allowing speech to reach the user's ear.

The instant invention solves the problem up to now existant, that of total attentuation of the noise and speech, by providing a solution of an active headset that can employ any of several selective algorithms such as those disclosed in US-patent 4 654 871. Alternatively, it can employ the algorithm disclosed in US-patent 5 105 377. In addition it can employ other algorithms such as that disclosed in US-patent 5 126 681.

Other objects, advantages and novel features of the present invention will become apparent from the following detailed description of the invention when considered in conjunction with the accompanying drawings.

Fig. 1 shows a typical active/passive headset system incorporating the instant invention.

Fig. 2 shows an active plus selective headset system with an open back muff that incorporates active control and adaptive speech filtering to allow speech to pass with the "anti-noise" signal.

Fig. 3 shows a more detailed description of the active control system of Figure 2.

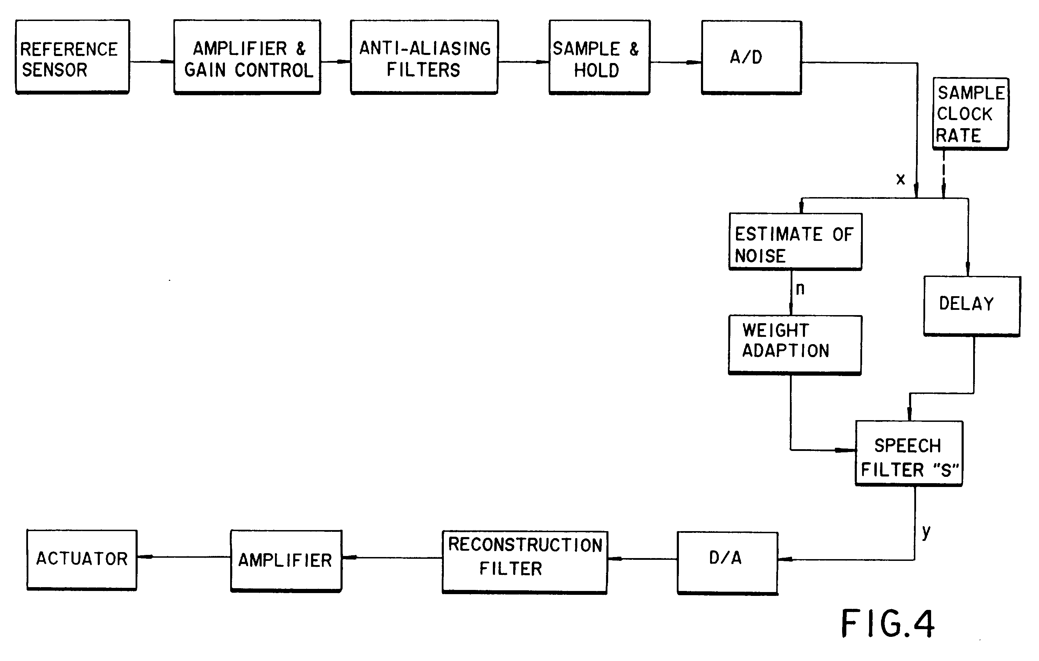

Fig. 4 shows a more detailed description of the adaptive speech filtering technique to be used in this headset design.

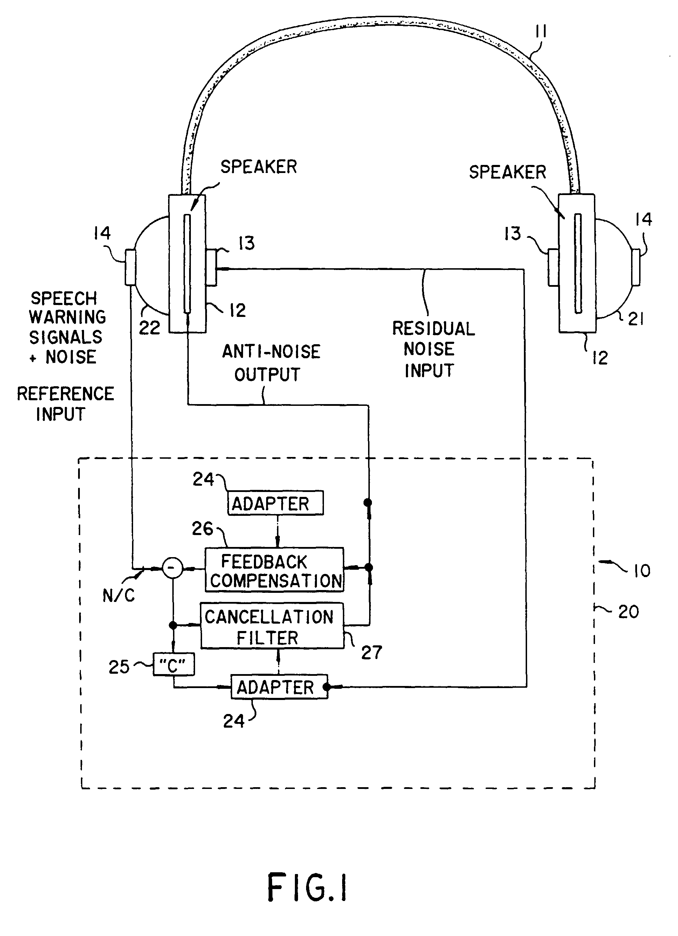

In Fig. 1 there is shown an active/passive closed back headset system 10. It consists of a typical passive headset 11, loudspeakers 12 that drive the anti-noise and residual microphones 13 to sense any remaining noise near the ear and reference microphones 14 to send advanced information for feed forward approaches and a system controller 20 which synthesizes the anti-noise signal.

The headset shown has closed backs 21, 22 for passive attenuation without the speakers, microphones and system controller, this headset would be a typical passive hearing protector.

The digital virtual earth (DVE) algorithm develops a reference signal by subtracting an equalized version of its own anti-noise signal from the residual signal. The adaptive feed forward uses the reference microphone as its input and is very effective on complicated noise environments that are broadband and random in character. The Least Means Square (LMS) adapter 24 shown in Fig. 1 are Filtered - X versions which have inherent compensation for the effects of the feedback delays around the loop. Box "C" at 25 is the impulse response of active cancellation system.

Feedback compensator 26 and cancellation filter 27 complete the component portions of the controller.

DVE is highly effective to use in simple noise environments having only a few harmonics even where the noise varies tremendously. It has also been demonstrated to be very effective doing broadband cancellation at low frequencies (50 - 700 Hz).

Speakers 12 of the headset are large enough to be capable of producing anti-noise at the same level as the noise to be canceled. They have little or no distortion and have a minimum of input-to-output delay as any delay in the feedback loop slows down the system adaptation rate.

Residual microphones 13 are typically small electret microphones mounted on the speaker frame near the ear. It must faithfully reproduce the sound that remains at the ear after cancellation so that the controller can make further adjustments to the anti-noise signal.

Reference microphones 14 are small electret microphones attached to the outside of the headset at a distance from the ear canal. This reference microphone is used to provide advanced information about the noise. The higher the frequency of the noise the more advanced information is needed to effectively cancel the noise.

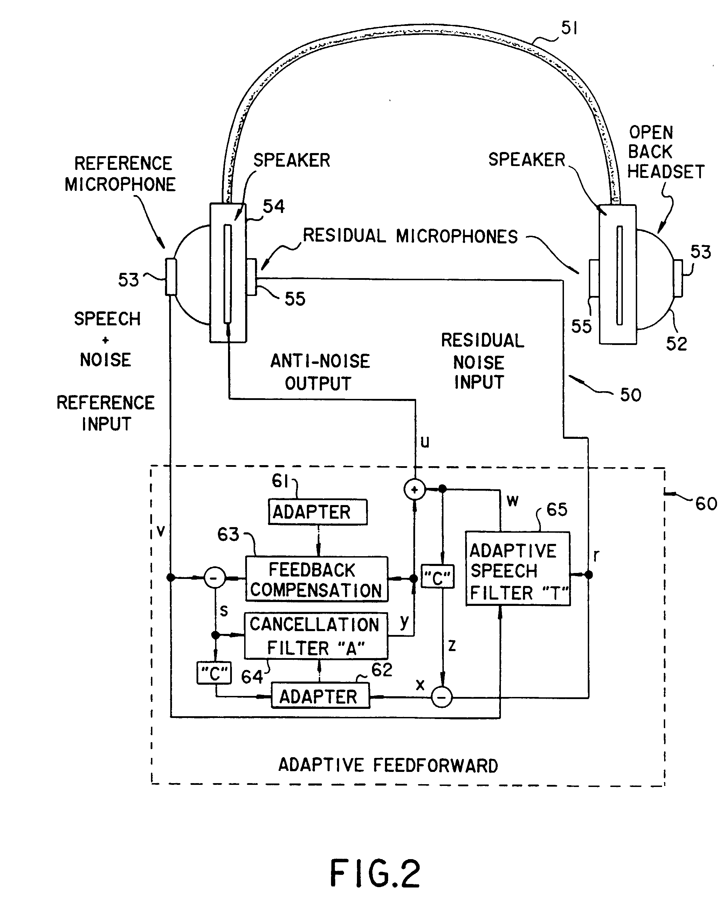

Fig. 2 shows an active plus selective headset system 50 with headset 51 having open backed muff positions 52, reference microphones 53, speakers 54 and residual microphones 55. An earplug (not shown) may be substituted for the open backed muff.

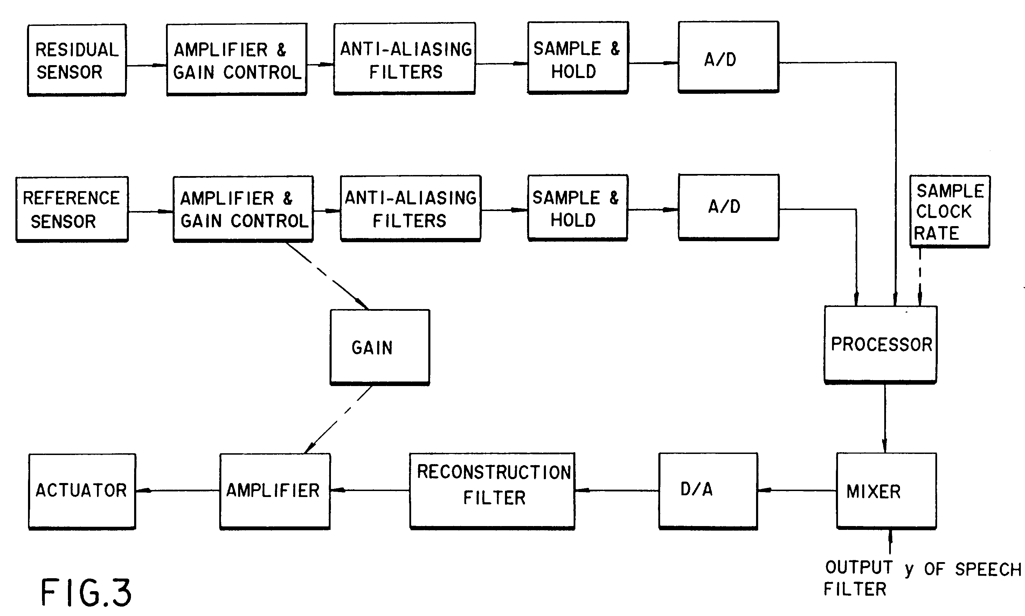

The active /passive system 10 previously described can be configured to actively attenuate all sounds in the frequency band from 20 to 3300 Hz without the need for a passive muff or earplug. The approach uses an adaptive feed forward control algorithm to actively attenuate the damaging noise in this band. In order to accomplish this it is necessary to minimize the delays of the digital signal processing system, which include delays introduced by the anti-aliasing and reconstruction filters shown in Figure 3 and the acoustic delay of the speaker and residual microphone physical system, in order to effectively attenuate noise at the higher frequencies.

The controller 60 has adapters 61,62, feedback compensation 63, cancellation filter 64 and adaptive speech filter 65. Controller 60 uses a parallel adaptive speech filtering technique to pass speech to the user. Adaptive speech filtering techniques can be employed to work with the particular noisy environment. The active controller attenuates noise in the band of interest and allows speech and warning signals to pass via the adaptive speech filtering path which incorporates a warning signal filter as shown in Fig. 2. It is similar to the active/passive system except for the open backed headset design and the addition of a parallel adaptive speech filtering path and warning signal filter path as integral parts of the controller. The input to the speech filter and controller are the upstream reference microphones 53.

This reference microphone contains noise and speech. The speech is filtered from the noise and passed with the "anti-noise" generated from the adaptive feed forward controller and sent to the headset loud speaker. Both speech and warning signals, which are typically above the speech band and of known frequencies, will be heard by the user of the lightweight and open back headset.

With reference to Figure 2, the "anti-noise" and speech output signals are mixed and input to the speakers. This combined signal output sample, $u_k$, is given by

$$\underline{x}_k = \underline{r}_k - \underline{z}_k$$

$$y_k = \underline{A}_k \underline{s}_k$$

$$u_k = w_k + y_k$$

$u_k$ is the output speech and anti-noise value
where $\underline{r}_k$ is a vector of the most recent examples of the residual signal
$\underline{z}_k$ is a vector of the output of the speech filter after it passed through the impulse response $\underline{C}_k$
$\underline{A}_k$ is a vector of cancellation filter coefficients
$y_k$ is the output anti-noise value
$w_k$ is the output speech value.
$\underline{s}_k$ is the vector of compensated inputs.

Inputs to the controller and speech filter are the reference signal, $v_k$, and residual signal $r_k$ that are picked up via the reference sensor and residual sensor respectively. The adaptive feedforward controller generates an "anti-noise", $y_k$, and the adaptive speech filter generates a clean speech signal. $w_k$, that are mixed to form the output signal $u_k$ which is sent to the speakers. Each ear piece operates independently with separate reference and residual sensors and actuator.

It is essential that the output of the speech filter, $w_k$, be filtered through the system in pulse response, $\underline{C}_k$, and subtracted from the residual input, $r_k$, so as not to interfere with the operation of the adaptive feedforward controller. Otherwise, the controller will attempt to adapt to and cancel the speech signal that is output to the speaker.

Several techniques can be used to minimize the delays of the system. First, passive material can effectively act as a low pass filter for the input reference and residual sensors. This would eliminate the need for anti-aliasing filters and thus the delays introduced by these filters would be eliminated. This technique has been shown to be quite effective in the active control of noise in ducts using the adaptive feedforward controller.

Another technique removes neither the anti-aliasing filters nor the reconstruction filters but essentially bypasses the delays introduced by these filters by inserting an analog zero'th order tap. This is achieved by placing an amplifier between the output of the incoming gain control and the output of the reconstruction filters shown in Figure 3.

A final technique, which will be even more effective as the speed of microprocessor technology increases, is to sample at a rate of 40 kHz or greater, this eliminates the need for anti-aliasing and reconstruction filters because the cut off frequency of 20 kHz is at the limit of the loudspeaker response.

## Claims

1. Headset system (10, 50) comprising:

    a headset means (11, 51) adapted to be worn by a user,
    means for attenuating noise and speech filtering means (27, 65)

    which by loudspeaker means (12, 54) transmits speech to the ears of the user,
    characterized by

    reference sensing means (14, 53) on said headset means (11, 51) adapted to sense speech, noise and warning signals and to generate signals input into a controller means (20, 60) adapted to generate and input signals into the loudspeaker means (12, 54) which causes active broadband attenuation of noise while selectively transmitting speech and warning signals to the ear of the user.

2. Headset system (10, 50) according to claim 1 characterized in that said headset system (10, 50) includes residual microphone means (13, 55) located on said headset means including a feedback means connected to said speaker means.

3. Headset system (10, 50) according to claim 2 characterized in that said feedback means includes an adaptive speech filter means (65) and a cancellation filter means (64).

4. Headset system (10, 50) according to claim 2 characterized in that said controller means is run by an adaptive feedforward algorithm.

5. Headset system (10, 50) according to claim 2, characterized in that said headset means (11, 51) includes an open backed muff (52).

6. Headset system (10, 50) according to claim 2, characterized in that said reference sensing means (14, 53) is an electret microphone.

7. Headset system (10,50) according to claim 6, characterized in that it comprises a pair of open backed muffs (52) with the reference sensing means (53) located on the outside of said muffs.

8. Headset system (10, 50) according to claim 1, characterized in that said adaptive speech filtering means (65) is adapted to filter speech from noise.

9. Headset system (10, 50) according to claim 1, characterized in that said adaptive speech filtering output (w) is mixed with said controller output (y) and the resulting signal (u) is passed to the speaker means.

10. Headset system (10, 50) according to claim 1, characterized in that said adaptive speech filtering output (w) is filtered by the impulse response of the active cancellation system ("C") and subtracted from the residual microphone signal (r) so as not to interfere with the operation of said controller.

11. Headset system (10, 50) according to claim 1 characterized in that said reference sensing means (14, 53) is external of speaker means on said headset means (11, 51) and said controller is adapted to employ a feedforward method of noise cancellation.

12. Headset system (10, 50) according to claim 1, characterized in that said reference sensing means (13, 55) is internal of said speaker means on said headset means (11, 51) and said controller is adapted to employ a feedback method of noise cancellation.

**Patentansprüche**

1. Kopfhörersystem (10, 50), mit einem Kopfhörer (11, 51), der geeignet ist, von einem Benutzer getragen zu werden, einem Mittel zur Abschwächung von Geräusch und einem Sprache filternden Mittel (27, 65), das durch Lautsprecher (12, 54) Sprache zu den Ohren des Benutzers überträgt,

**gekennzeichnet, durch**
ein eine Referenz sensierendes Mittel (14, 53) auf dem Kopfhörer (11, 51), das geeignet ist, Sprache, Geräusch und Warnsignale zu sensieren und Signale zu erzeugen, die in ein Mittel zum Steuern (20, 60) eingegeben werden, das dazu dient, Signale für die Lautsprecher (12, 54) zu erzeugen und diesen zuzuführen, was aktive Breitbanddämpfung von Geräusch bewirkt, während selektiv Sprache und Warnsignale zum Ohr des Benutzers übertragen werden.

2. Kopfhörersystem (10, 50) gemäß Anspruch 1, dadurch gekennzeichnet, daß das Kopfhörersystem (10, 50) Rest-Mikrophone (13, 55), die auf dem Kopfhörer angeordnet sind, einschließlich einem Rückkopplungsmittel, das mit den Lautsprechern verbunden ist, umfaßt.

3. Kopfhörersystem (10, 50) gemäß Anspruch 2, dadurch gekennzeichnet, daß das Rückkopplungsmittel ein anpassungsfähiges Sprache-Filter-Mittel (65) und ein Auslöschungs-Filter-Mittel (64) einschließt.

4. Kopfhörersystem (10, 50) gemäß Anspruch 2, dadurch gekennzeichnet, daß das Mittel zum Steuern mit einem "adaptive feedforward"-Algorithmus betrieben wird.

5. Kopfhörersystem (10, 50) gemäß Anspruch 2, dadurch gekennzeichnet, daß der Kopfhörer (11, 51) eine Hörmuschel (52) mit offener Rückseite einschließt.

6. Kopfhörersystem (10, 50) gemäß Anspruch 2, dadurch gekennzeichnet, daß das eine Referenz sensierende Mittel (14, 53) ein Elektret-Mikrophon ist.

7. Kopfhörersystem (10, 50) gemäß Anspruch 6, dadurch gekennzeichnet, daß es ein Paar Hörmuscheln (52) mit offener Rückseite umfaßt, wobei die eine Referenz sensierenden Mittel (53) auf der Außenseite dieser Hörmuscheln angeordnet sind.

8. Kopfhörersystem (10, 50) gemäß Anspruch 1, dadurch gekennzeichnet, daß das anpassungsfähige, Sprache filternde Mittel (65) geeignet ist, Sprache aus Geräusch zu filtern.

9. Kopfhörersystem (10, 50) nach Anspruch 1, dadurch gekennzeichnet, daß das anpassungsfähige, Sprache filternde Ausgangssignal (w) mit dem Ausgangssignal (y) des Mittels zum Steuern gemischt wird und das sich ergebende Signal (u) dem Lautsprecher zugeführt wird.

10. Kopfhörersystem (10, 50) gemäß Anspruch 1, dadurch gekennzeichnet, daß das anpassungsfähige, Sprache filternde Ausgangssignal (w) durch die Impulsantwort des aktiven Auslöschungssystems ("C") gefiltert wird und von dem Restmikrophonsignal (r) subtrahiert wird, so daß es nicht den Betrieb des Steuermittels beeinflußt.

11. Kopfhörersystem (10, 50) gemäß Anspruch 1, dadurch gekennzeichnet, daß das eine Referenz sensierende Mittel (14, 53) sich außerhalb von Lautsprechern auf dem Kopfhörer (11, 51) befindet und das Mittel zum Steuern geeignet ist, eine "feedforward"-Methode der Geräuschauslöschung zu verwenden.

12. Kopfhörersystem (10, 50) gemäß Anspruch 1, dadurch gekennzeichnet, daß das eine Referenz sensierende Mittel (13, 55) sich innerhalb der Lautsprecher auf dem Kopfhörer (11, 51) befindet und das Mittel zum Steuern geeignet ist, eine Rückkopplungs-Methode ("feedback"-Methode) der Geräuschauslöschung zu verwenden.

**Revendications**

1. Système de casque d'écoute (10, 50) comprenant :

   un moyen de casque d'écoute (11, 51) conçu pour être porté par un utilisateur, un moyen destiné à atténuer le bruit et un moyen de filtrage de la parole (27, 65),

   lequel, grâce à un moyen d'écouteur (12, 54) transmet la parole vers les oreilles de l'utilisateur, caractérisé par un moyen de détection de référence (14, 53) sur ledit moyen de casque d'écoute (11, 51), conçu pour détecter des signaux de parole, de bruit et d'avertissement et pour générer des signaux reçus en entrée dans un moyen de contrôleur (20, 60) conçu pour générer et appliquer en entrée des signaux dans le moyen d'écouteur (12, 54) qui provoque une atténuation à large bande active du bruit tout en transmettant sélectivement la parole et les signaux d'avertissement vers l'oreille de l'utilisateur.

2. Système de casque d'écoute (10, 50), selon la revendication 1, caractérisé en ce que ledit système de casque d'écoute (10, 50) comprend un moyen de microphone résiduel (13, 55) situé sur ledit moyen de casque d'écoute comprenant un moyen de contre-réaction relié audit moyen d'écouteur.

3. Système de casque d'écoute (10, 50) selon la revendication 2, caractérisé en ce que ledit moyen de contre-réaction comprend un moyen de filtre vocal adaptatif (65) et un moyen de filtre d'annulation (64).

4. Système de casque d'écoute (10, 50) selon la revendication 2, caractérisé en ce que ledit moyen de contrôleur est mis en oeuvre grâce à un algorithme de pré-compensation adaptatif.

5. Système de casque d'écoute (10, 50) selon la revendication 2, caractérisé en ce que ledit moyen de casque d'écoute (11, 51) comprend un manchon à dos ouvert (52).

6. Système de casque d'écoute (10, 50) selon la revendication 2, caractérisé en ce que ledit moyen de détection de référence (14, 53) est un microphone à électret.

7. Système de casque d'écoute (10, 50) selon la revendication 6, caractérisé en ce qu'il comprend une paire de manchons à dos ouvert (52), le moyen de détection de référence (53) étant situé sur l'extérieur desdits manchons.

8. Système de casque d'écoute (10, 50) selon la revendication 1, caractérisé en ce que ledit moyen de filtrage adaptatif de la parole (65) est conçu pour séparer la parole du bruit.

9. Système de casque d'écoute (10, 50) selon la revendication 1, caractérisé en ce que ladite sortie de filtrage adaptatif de la parole (w) est mélangée avec ladite sortie du contrôleur (y), et en ce que le signal résultant (u) est transmis au moyen d'écouteur.

10. Système de casque d'écoute (10, 50) selon la revendication 1, caractérisé en ce que ladite sortie de filtrage adaptatif de la parole (w) est filtrée par la réponse impulsionnelle du système d'annulation actif ("C") et puis soustraite du signal résiduel du microphone (r) de façon à ne pas interférer avec le fonctionnement dudit contrôleur.

11. Système de casque d'écoute (10, 50) selon la revendication 1, caractérisé en ce que ledit moyen de détection de référence (14, 53) est externe au moyen d'écouteur sur ledit moyen de casque d'écoute (11, 51), et en ce que ledit contrôleur est conçu pour utiliser un procédé d'annulation du bruit par pré-compensation

**12.** Système de casque d'écoute (10, 50) selon la revendication 1, caractérisé en ce que ledit moyen de détection de référence (13, 55) est interne audit moyen d'écouteur sur ledit moyen de casque d'écoute (11, 51), et en ce que ledit contrôleur est conçu pour utiliser un procédé d'annulation du bruit par contre-réaction.

EP 0 643 881 B1

FIG.1

7

FIG.2

FIG.3

EP 0 643 881 B1

EP 0 643 881 B1

REFERENCE SENSOR → AMPLIFIER & GAIN CONTROL → ANTI-ALIASING FILTERS → SAMPLE & HOLD → A/D

SAMPLE CLOCK RATE

x

ESTIMATE OF NOISE

n

WEIGHT ADAPTION

DELAY

SPEECH FILTER "S"

y

ACTUATOR ← AMPLIFIER ← RECONSTRUCTION FILTER ← D/A

FIG.4